# EUROPÄISCHE PATENTSCHRIFT

(11) **EP 1 411 863 B1**
(45) Veröffentlichungstag und Bekanntmachung des Hinweises auf die Patenterteilung: **10.12.2008**
(21) Anmeldenummer: 02754298.4
(22) Anmeldetag: 25.06.2002
(51) Int. Cl.: A61F 2/06

(54) **UMMANTELUNG FÜR VENEN**
COVERING ELEMENT FOR VEINS
GAINE POUR VEINES

(30) Priorität: 31.07.2001 DE 10137414
(43) Veröffentlichungstag der Anmeldung: 28.04.2004
(73) Patentinhaber: Aesculap AG, 78532 Tuttlingen (DE)
(72) Erfinder: MORITZ, Anton, 60590 Frankfurt (DE); GOLDMANN, Helmut, 78532 Tuttlingen (DE); KREUZ, Patricia, 66113 Saarbrücken (DE)
(74) Vertreter: Patentanwälte Ruff, Wilhelm, Beier, Dauster & Partner
(86) Internationale Anmeldenummer: PCT/DE2002/002391
(87) Internationale Veröffentlichungsnummer: WO 2003/011190

(56) Entgegenhaltungen:
- EP-A- 0 327 903
- WO-A-01/89594
- WO-A-99/40875
- US-A- 6 136 022
- US-A1- 2001 012 962

## Beschreibung

Die vorliegende Erfindung betrifft eine Ummantelung für Venen.

Die Medizin steht häufig vor der Aufgabe der Behandlung von cardiovaskulären Erkrankungen, die durch Veränderungen an Blutgefäßen wie beispielsweise Arteriosklerose bedingt sind. Hierzu werden in der modernen Chirurgie, neben z. B. Desobliterationsverfahren, Gefäßersatzimplantate in Form von sogenannten Bypässen zur Rekonstruktion arterieller Gefäße eingesetzt. Bekannt sind beispielsweise Gefäßimplantate aus synthetischen Materialien oder aus synthetischen Materialien kombiniert mit natürlichen Materialien.

Beim Einsatz von vollsynthetischen Materialien kann es zu nachteiligen Wechselwirkungen mit der physiologischen Umgebung im Patientenkörper kommen. Besonders beim Ersatz kleinlumiger koronarer und peripherer Gefäße mit synthetischem Material können schwerwiegende Komplikationen, wie beispielsweise Thrombusbildung und Restenosierung des Gefäßimplantates auftreten, die kostenintensive Nachoperationen erfordern können. Wünschenswert ist deshalb die Verwendung von Implantaten, die aus natürlichem Gefäßmaterial gebildet sind, da sich die natürliche Endothelialisierung und die antithrombogenen Eigenschaften der Gefäßwand vorteilhaft auswirken. Im allgemeinen werden solche biologischen Gefäßersatzmaterialien aus Venen gewonnen. Bei der Implantation von Venen in das arterielle Gefäßsystem kommt es im Zuge der Arterialisierung zu einer Zunahme der Wanddicke. Ist dies aufgrund der unterschiedlichen Compliance zwischen Vene und Arterie begleitet durch eine Intimahyperplasie kann dies schließlich zur Restenosierung des Implantates führen. Damit native Venen ihre vorgesehene Funktion, beispielsweise als koronarer oder peripherer Bypass zum arteriellen Bluttransport, dauerhaft und zuverlässig erfüllen können, ist es vorteilhaft, sie extern zu ummanteln oder zu verstärken. Durch eine externe Ummantelung oder Schienung kann die Compliance des naturlichen Venenmaterials an das arterielle System angepaßt und damit die Intimahyperplasie reduziert und langfristig eine gute Offenheitsrate erreicht werden. Desweiteren ermöglicht die beschriebene Ummantelung auch die Implantation von varikösen, ektatischen und dünnwandigen Venen, die bislang aufgrund ihrer ungünstigen hämodynamischen Eigenschaften nicht verwendet wurden. Dies ist besonders bei Patienten mit multiplen Gefäßdefekten, bei denen sonst kein geeignetes Venenmaterial für den Ersatz kleinlumiger Gefäße zur Verfügung steht, die einzige Möglichkeit zur Verwendung körpereigener Gefäßmaterialien.

Bekannt ist aus DE 4340755 eine Gefäßprothese, bei der ein natürliches Blutgefäß mit einem Geflecht überzogen ist. In EP 687164 ist ein natürliches Blutgefäß von sich kreuzenden Fäden wendelförmig umwunden. US 5,645,581 offenbart einen Schlauch mit spiralförmig um die Längsachse gewundenen sich kreuzenden Fäden, dessen Herstellungsverfahren in US 5,755,659 beschrieben ist.

Gemäß DE 19910340 wird ein Schlauch, ein Mantel oder eine Röhre als Ummantelung einer Vene für Arterienprothesen verwendet.
Aus WO 00/54703 ist eine äussere Verstärkung für Gefäßprothesen mit einer doppelten Polymerfaserschlauchumhüllung bekannt.
Die WO-A-01/89594 (Stand der Technik gemäß Art. 54(3)) offenbart eine implantierbare Prothese deren Maschenware gestrickt oder gewirkt sein kann.

Die bekannten Verstärkungen für Gefäßmaterial weisen eine Reihe von Nachteilen auf. So sind zahlreiche aufwändige Arbeitsschritte erforderlich, um sie vorzubereiten und am Implantatgefäß anzubringen. Es sind zusätzlich Gewebekleber erforderlich, um die Verstärkung zu befestigen.

Verstärkungen auf Basis von Metallen können die Handhabbarkeit der Prothese beeinträchtigen und Unverträglichkeitsreaktionen begünstigen.
Durch freie Drahtenden können besonders im Anastomosenbereich Probleme auftreten.

Die Erfindung stellt sich daher die Aufgabe, eine Ummantelung für Venen zur Verfügung zu stellen, die die Probleme aus dem Stand der Technik überwindet, die die Venen für die Verwendung als chirurgisches Implantat zum dauerhaften Gefäßersatz verstärkt, die einfach und kostengünstig nach üblichen Verfahrenstechniken und auf üblichen Produktionsseinrichtungen hergestellt werden kann, und die in der Chirurgie einfach und zuverlässig anwendbar ist.

Diese Aufgabe wird gelöst durch eine Ummantelung gemäß Anspruch 1.

Die vorliegende Erfindung ist besonders geeignet zur Verwendung als Ummantelung für die Verstärkung von nativen Venen zur Bereitstellung eines chirurgischen Implantats als Gefäßersatz in der Humanmedizin und Veterinärmedizin.

Die Venenummantelung kann mit Vorteil als offenporiger textiler Schlauch durch Wirken hergestellt sein. In einer Ausführungsform kann das Gewirke auf üblichen Rundwirkmaschinen für kleinlumige Schläuche hergestellt sein. In einer anderen Ausführungsform kann zur Herstellung der Gewirke eine doppelbarrige Raschelmaschine verwendet sein. Die Verfahrenstechnik des Wirkens ist den Fachleuten allgemein bekannt und soll deshalb hier nicht ausführlich erläutert werden.

In einer Ausführungsform kann die erfindungsgemäße Venenummantelung durch Rundwirken in Einfach-Trikotbindung ausgebildet sein. In einer bevorzugten Ausführungsform kann die erfindungsgemäße Venenummantelung durch Wirken in einer Kombination von Bindungstechniken ausgebildet sein. Als Beispiele solcher kombinierter Wirktechniken sind die Bindungen Trikot-Atlas, Trikot-Franse, Franse/Schuss und Kombinationen mit Fileteinzug zu nennen. Ein Gewirk in Trikot-Atlasbindung ist für die Ummantelung von Venen gemäß der Erfindung bevorzugt. Es können noch verschiedene weitere Varianten und Wirkkombinationen ausgeführt sein, wie beispielsweise offene Maschen oder geschlossene Maschen.

Die polygonen Öffnungen im Netz können in Abhängigkeit von der gewählten Wirktechnik unterschiedliche Form aufweisen. In einer Ausführungsform können die Netzöffnungen rautenförmig ausgebildet sein. Insbesondere bei Gewirken in Einfach-Trikotbindung können rautenförmige Durchbrüche gebildet sein. Der lichte Durchmesser der Rauten kann insbesondere im Bereich von 100 bis 600 µm, insbesondere im Bereich von 100 bis 400 µm oder falls größere Öffnungen gewünscht sind bevorzugt im Bereich von 300 bis 600 µm liegen. Bei Gewirken in Atlas-Trikotbindung können durch Abrundung von Ecken, insbesondere wabenförmige Durchbrüche gebildet sein. Der lichte Durchmesser der Durchbrüche kann bevorzugt im Bereich von 400 bis 1600 µm, insbesondere von 800 bis 1200 µm, besonders bevorzugt von 600 bis 1000 µm liegen.

Mit besonderem Vorteil können die schlauchförmigen Gewirke im wesentlichen aus biokompatiblen Polymerfasern gebildet sein. Als Beispiele für solche biokompatiblen Polymere sind synthetische Polymere in Form von Homopolymeren, Copolymeren, Terpolymeren oder Polymerblends, natürliche Polymere oder Kombinationen von synthetischen und natürlichen Polymeren zu nennen. Die Verwendung von synthetischen resorbierbaren Polymeren ist ebenfalls denkbar. In einer bevorzugten Ausführungsform der erfindungsgemäßen Venenummantelung ist ein hochkapillares Polyestergarn aus Polyethylenterephthalat (PET) verwendet. PET zeichnet sich durch gute Biokompatibilität aus und ist daher als Implantatmaterial besonders geeignet.

Gemäß der Erfindung weist die Ummantelung für Venen im wesentlichen keinen Velour auf. Das Gewirk kann sich auf diese Weise durch im wesentlichen glatte Oberflächen auf der Außenseite und auf der Innenseite auszeichnen. In einer besonderen Ausführungsform kann die Ummantelung im wesentlichen frei von texturierten Fäden ausgebildet sein.

Mit Vorteil kann die Venenummantelung aus Multifilamentgarn gebildet sein. Die erfindungsgemäße Ummantelung kann aus Garn mit 2 bis 500 Filamenten, insbesondere 5 bis 250 Filamenten, bevorzugt 10 bis 100 Filamenten gebildet sein. Das erfindungsgemäß verwendete Garn kann sich durch eine Feinheit von vorzugsweise 50f40 dtex auszeichnen.
Das Gewirk der erfindungsgemäßen Ummantelung kann mit einer Maschenweite von 100 bis 1000 µm, insbesondere 300 bis 600 µm ausgebildet sein.

Bei der Einfach-Trikotbindung, auch Single-Trikotbindung genannt, wird mit nur einer Legeschiene gewirkt. Durch geeignete Wahl der Fadenzahl im Garn, der Reihendichte und Stäbchendichte des Gewirkes kann der Innendurchmesser des Gewirkschlauches für die Ummantelung von Venen nach Wunsch eingestellt werden. Bei Ummantelungsschläuchen in Einfachbindung, insbesondere Einfach-Trikotbindung können die Fadenzahlen mit Vorteil bei 5 bis 25, die Reihendichten pro Zentimeter bei 10 bis 20, die Stäbchendichte pro Zentimeter bei 15 bis 25 und die Nenndurchmesser bei 2 bis 10 mm liegen. Insbesondere bei der Einfach-Trikotbindung ergeben sich Gewirke mit geringerer Wanddicke. Bevorzugt können Wanddicken von Einfach-Wirkschläuchen, Einfach-Trikotschläuchen im Bereich von 0,10 bis 0,25 mm liegen.

Bei der kombinierten Wirktechnik, insbesondere in Trikot-Atlasbindung wird mit zwei Legeschienen und besonderem Fadeneinzug gewirkt. Der erhaltene Gewirkschlauch weist insbesondere eine wabenähnliche Struktur auf. Um eine losere Maschenstruktur zu erreichen, kann ein Garn mit geringerer Fadenzahl verwendet werden. Durch geeignete Wahl der Fadenzahl im Garn, der Reihendichte und Stäbchendichte des Gewirkes kann der Innendurchmesser des Gewirkschlauches für die Ummantelung von Venen nach Wunsch eingestellt werden. Bei Ummantelungsschläuchen in kombinierter Wirktechnik, insbesondere in Trikot-Atlasbindung, können die Fadenzahlen mit Vorteil bei 15 bis 90, die Reihendichten pro Zentimeter bei 20 bis 40, die Stäbchendichte pro Zentimeter bei 20 bis 30 und die Nenndurchmesser bei 2 bis 15 mm liegen. Bevorzugt können Wanddicken im Bereich von 0,10 bis 0,30 mm, insbesondere bei 0,15 bis 0,25 mm liegen. Bevorzugt kann erfindungsgemäß ein Garn der Feinheit 50f40 dtex verwendet sein.

Durch die intensivere Verschlingung der Garnstränge in der kombinierten Wirktechnik werden hier im Vergleich zum Einfach-Trikot bei der kombinierten Wirktechnik stabilere Stäbchen und Reihen erhalten. Ein solches Gewirk kann sich durch geringere Dehnbarkeit und größere Verschiebefestigkeit auszeichnen. Auf diese Weise sind Gewirkschläuche mit geringerer Aufweitbarkeit im Lumen erhältlich. Auf diese Weise sind erfindungsgemäße Gewirke beständiger gegen Belastungen durch arteriellen Blutdruck.

Strukturmerkmale der nach den als bevorzugte Ausführungsformen beschriebenen Wirktechniken in Einfach-Trikotbindung und Trikot-Atlasbindung hergestellten Gewirke für Venenummantelungen werden aus den beigefügten Abbildungen von mikroskopischen Aufnahmen noch deutlicher ersichtlich.

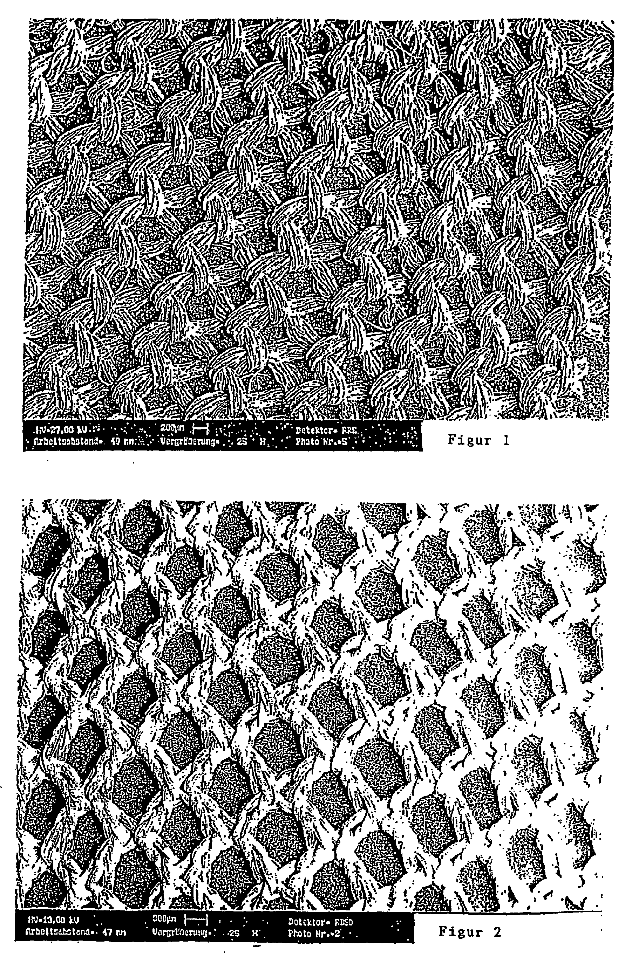

Figur 1 zeigt ein Einfach-Trikotgewirk in 25facher Vergrößerung. Die im Gewirk ausgebildeten Netzöffnungen sind annähernd rautenförmig bis quadratisch mit lichten Weiten im Bereich von etwa 300 bis 600 µm. In den Reihen und Stäbchen sind die Garnstränge nur einfach umeinander geschlungen, was eine gewisse Verschiebbarkeit der Maschen und Dehnung des Gewirkes ermöglicht.

Figur 2 zeigt ein Trikot-Atlasgewirk in 25facher Vergrößerung. Die im Gewirk ausgebildeten Netzöffnungen sind annähernd wabenförmig bis rechteckig mit lichten Weiten im Bereich von 400 bis 1200 µm, insbesondere 600 bis 800 µm. Auch in den Reihen und Stäbchen sind die Garnstränge miteinander verschlungen, so dass sich eine größere Stabilität gegen Verschiebung der Maschen ergibt.

Ein durch die oben beschriebenen Wirktechniken erhaltener Gewirkschlauch kann für die Umhüllung einer Vene in geeigneter Weise vorbehandelt sein. In einer Ausführungform der Erfindung kann das Rohgewirk durch Reinigen vorbehandelt sein. In einer anderen Ausführungsform der Erfindung kann das Rohgewirk durch thermisches Schrumpfen und Reinigen vorbehandelt sein.

Das Reinigen des rohen Gewirkschlauches kann in drei Schritten vorgenommen werden. Zunächst wird das Material in 60 °C warmes Wasser eingelegt und gerührt. Danach wird es in einer Extraktionsapparatur über Isopropanol extrahiert, wobei auch Avivagereste entfernt werden. Schliesslich wird das Gewirk nochmals in warmem Wasser von 40 °C nachbehandelt. Nach der Reinigung wird der Gewirkschlauch in geeigneter Weise getrocknet, wie beispielsweise im Laminarflow.

In einer anderen Ausführungsform kann das rohe Gewirk zusätzlich durch Schrumpfen vorbehandelt werden. Hierbei wird eine Schrumpfbehandlung vor der oben beschriebenen Reinigung vorgenommen. Das Schrumpfen kann durch Eintauchen und Verweilen des Gewirkschlauches in siedendem Wasser durchgeführt werden.

Zur thermischen Fixierung kann gereinigtes und gegebenenfalls geschrumpftes schlauchförmiges Gewirk auf einen Metallstab aufgezogen, ein beiden Enden arretiert und bei 160 °C im Trockenschrank thermisch fixiert werden. Dabei werden die Gewirkschläuche ausgehend vom deklarierten Innendurchmesser des Rohmaterials unterschiedlich stark aufgeweitet, was in den folgenden Beispielen 4 und 5 ausführlicher erläutert wird. Die Thermofixierung kann in einem Schritt durchgeführt werden. Alternativ kann die Thermofixierung in zwei Schritten durchgeführt werden, wobei die Gewirkschläuche stärker aufgeweitet werden.

Gewirkte Schläuche werden vorzugsweise unabhängig von einer Vorbehandlung durch Schrumpfen thermofixiert. Mit anderen Worten, die Verfahrensweise der Thermofixierung ist nicht durch die Art der Vorbehandlung durch Schrumpfen bestimmt. Da die rohrförmigen Gewirke während der thermischen Fixierung auf den Metalldornen beidseitig fixiert sind, können sie danach nicht mehr so stark schrumpfen. Das führt bei nicht vorgeschrumpftem Gewirk im Vergleich zu geschrumpftem Gewirk zu größeren Poren. Die erfindungsgemäße Ummantelung kann sich vorteilhaft durch weitgehende Formstabilität auszeichnen.

Nach der Thermofixierung und der Abkühlung des Metallstabes auf Raumtemperatur werden die Arretierungen entfernt, die Venenummantelung auf eine Länge von etwa 10 bis 60 cm, bevorzugt 10 bis 30 cm zugeschnitten und verpackt.

Bestimmungen der normierten radialen Reißkraft von verschiedenen Proben in Einfach-Trikotbindung und kombinierten Wirktechniken, wie Trikot-Atlasbindung, zeigen in Abhängigkeit von Wirkart und Behandlung der Gewirkschläuche Reißkraftwerte im Bereich von 2 bis 10 N/mm, insbesondere 2 bis 6 N/mm. Die radiale Reißfestigkeit der Gewirkschläuche ohne Schrumpfbehandlung ist geringer als die der Gewirkschläuche mit Schrumpfbehandlung.

Messungen der longitudinalen Reißkraft bei Einfach-Trikotgewirken ergeben Werte zwischen 70 und 100 N für Proben mit und ohne Schrumpfbehandlung.

Das zugelastische Verhalten der Venenummantelung wurde in radialer Richtung in einem Kraftbereich von 2 bis 12 N bestimmt. Für Proben in Einfach-Trikotbindung und kombinierter Wirktechnik werden in radialer Richtung elastische Dehnungswerte im Bereich von 3 bis 10 %, insbesondere 5 bis 8 % bei einer plastischen Dehnung in der gleichen Größenordnung zwischen 5 bis 15 %, insbesondere 6 bis 13 % ermittelt.

Zur Bereitstellung für die Chirurgie können die Ummantelungen für Venen gemäß der Erfindung in zweckmäßiger Länge zugeschnitten gebrauchsfertig in geeigneter Weise verpackt vorliegen. Insbesondere kann das erfindungsgemäße Ummantelungsmaterial in geeigneter Weise sterilisiert werden. Ein zweckmäßiges Sterilisierverfahren kann aus üblichen physikalischen oder chemischen Methoden zum Inaktivieren von Mikroorganismen ausgewählt oder eine Kombination solcher Methoden sein. Ein mögliches Sterilisierungsverfahren umfasst die Behandlung mit Ethylenoxid. Bevorzugt kann eine Sterilisierung der erfindungsgemäßen Ummantelung unter Verwendung von γ-Strahlung vorgenommen werden.

Bei dem mit der erfindungsgemäßen Ummantelung zu versehenden Venenmaterial handelt es sich um eine natürliche aus einem Säuger entnommene Vene. Eine solche Vene oder ein Venenteilstück kann einem toten Spender entnommen sein. Alternativ kann eine solche Vene oder ein Venenteilstück einem noch lebenden Spender entnommen sein. Der Venenspender kann ein Tier sein, beispielsweise ein Schwein. Der Venenspender kann ein Mensch sein. Besonders bevorzugt kann die zu ummantelnde Vene dem Patienten selbst entnommen sein, der das ummantelte Gefäßimplantat erhalten soll. Eine solche Ausführungsform ist besonders vorteilhaft, da auf diese Weise eine problemlose Einheilung körpereigenen Gewebes zu erwarten ist und sich Unverträglichkeitsreaktionen auf das Implantat minimieren lassen. Bei der Ummantelung einer natürlichen Vene gemäß der Erfindung ist keine zusätzliche Fixierung der Ummantelung mit Gewebekleber erforderlich. Auf diese Weise kann der technische Aufwand bei der Implantation und das Risiko von Komplikationen weiter verringert werden.

Im Folgenden wird die vorliegende Erfindung durch ausführliche Beschreibung von besonderen Ausführungsformen in Form von Beispielen weiter erläutert. In den Beispielen können einzelne Merkmale der Erfindung allein oder in Kombination mit anderen Merkmalen verwirklicht sein. Die Beispiele dienen nur der Erläuterung und besseren Verständlichkeit der Erfindung und sind in keiner Weise als Einschränkung zu verstehen.

### Beispiel 1

### Herstellung eines Gewirkschlauches

Als Rohmaterial für die Venenummantelung wird ein Gewirk in Trikot-Atlasbindung mit zwei Legeschienen hergestellt. Es wird Polyethylenterephthalatfasergarn in einer Feinheit von 50f40 dtex mit einer Reihendichte von etwa 35 pro Zentimeter und einer Stäbchendichte von etwa 25 pro Zentimeter verarbeitet. Bei einer Fadenzahl von 20 ergibt sich ein Gewirkschlauch mit einem Nenndurchmesser von 4 mm. Bei einer Fadenzahl von 80 ergibt sich ein Gewirkschlauch mit einem Nenndurchmesser von 14 mm.

### Beispiel 2

### Vorbehandlung durch Reinigen

Das in Beispiel 1 erhaltene Rohgewirk wird ohne Schrumpfen in einem ersten Schritt für 30 min unter Rühren in 60 °C warmen demineralisiertem Wasser gereinigt. In einem zweiten Schritt wird das Material in einer Soxhlet-Appratur über Isopropanol während eines von der Materialmenge abhängigen Zeitraums von15 min bis 3 h gereinigt und extrahiert. Dabei werden Reste von Avivage entfernt. In einem dritten Reinigungsschritt wird das Material erneut in 40 °C warmem Wasser für 10 min unter ständigem Rühren inkubiert. In einem letzten Schritt wird das gereinigte Material über Nacht im Laminarflow getrocknet.

### Beispiel 3

### Vorbehandlung durch Schrumpfen

Das in Beispiel 1 erhaltene Rohgewirk wird 5 min bei 97 bis 100 °C in siedendem demineralisiertem Wasser geschrumpft. Danach wird das geschrumpfte Material in einem ersten Schritt für 30 min unter Rühren in 60 °C warmen demineralisiertem Wasser gereinigt. In einem zweiten Schritt wird das Material in einer Soxhlet-Apparatur über Isopropanol während eines von der Materialmenge abhängigen Zeitraums von 15 min bis 3 h gereinigt und extrahiert. Dabei werden Reste von Avivage entfernt. In einem dritten Reinigungsschritt wird das Material erneut in 40 °C warmem Wasser für 10 min unter ständigem Rühren inkubiert. In einem letzten Schritt wird das geschrumpfte und gereinigte Material über Nacht im Laminarflow getrocknet.

### Beispiel 4

### Thermofixieren von Einfach-Trikotgewirken

Gemäß Beispiel 3 vorbehandelte röhrenförmige Gewirk mit einem deklarierten Innendurchmesser von 3 mm wird auf eine Länge von 40 cm zugeschnitten, auf einen Metallstab mit einem Außendurchmesser von 6 mm aufgezogen und in einem Schritt thermisch fixiert. In gleicher Weise werden Gewirke mit einem deklarierten Innendurchmesser von 4 und 5 mm auf einen Stab mit einem Außendurchmesser von 7 bzw. 8 mm aufgezogen und thermofixiert. Der endgültige Innendurchmesser der Venenummantelung entspricht dabei dem Außendurchmesser des verwendeten Metalldorns.

Bei einer zweistufigen Thermofixierung werden Schläuche mit einem deklarierten Innendurchmesser von 3 mm in einem ersten Fixierungsschritt auf einen Metallstab mit einem Außendurchmesser von 5 mm und im zweiten Fixierungsschritt auf einen Dorn mit einem Außendurchmesser von 6 mm aufgezogen und nach jedem Schritt thermisch fixiert. Nach dem gleichen Verfahren werden Schläuche mit einem deklarierten Innendurchmesser von 4 mm auf 7 und 8 mm aufgeweitet und fixiert.

### Beispiel 5

### Thermofixieren von Trikot-Atlasgewirken

Im Gegensatz zu den in Beispiel 4 beschriebenen Einfach-Trikotgewirken lassen sich die Trikot-Atlasgewirke nur geringfügig aufweiten. Ein solches T,rikot-Atlasgewirk mit einem deklarierten Innendurchmesser von 7 mm wird bei einstufiger Thermofixierung auf Metallstäbe mit Außendurchmessern von 6, 7 oder 8 mm aufgezogen und thermisch fixiert. Es können gereinigte und zusätzlich geschrumpfte Gewirke gleichermassen behandelt werden. Bei zweistufiger Thermofixeriung wird ein Trikot-Atlasgewirk mit einem deklarierten Innendurchmesser von 7 mm zunächst auf einen Metallstab mit einem Außendurchmesser von 7 mm thermisch fixiert und nach dem Abkühlen auf einem weiteren Metalldorn mit einem Außendurchmesser von 8 mm ein zweites Mal thermisch fixiert.

### Beispiel 6

### Eigenschaften der Venenummantelung

Bei den gemäß den oben beschriebenen Beispielen hergestellten Venenummantelungen wurden geometrische und physikalische Eigenschaften bestimmt.

Die mittlere Wandstärke einer in Einfach-Trikotbindung ausgeführten Ummantelung liegt bei 0,17 +/- 0,01 mm.
Die mittlere Wandstärke einer in Trikot-Atlasbindung ausgeführten Ummantelung liegt bei 0,22 bis 0,23 +/- 0,01 mm.

Messungen der radialen Reißkraft zeigen eine deutliche Abhängigkeit der Werte von der Art der Vorbehandlung. Durch Kochschrumpfbehandlung kann die Reißfestigkeit z. T. beträchtlich erhöht werden.

An exemplarischen Proben ermittelte Reißkraftwerte sind in der folgenden Tabelle 1 angegeben:

**Tabelle I**

| Gewirkart | Herstellungsverfahren | Radiale Reißkraft (normiert) |
|---|---|---|
| | | (N/mm) |
| Einfach-Trikot | Durchmesser | 4,5 +/-1,6 |
| | von 4 auf 8 mm | n = 13 |
| | (zweistufig 4 → 7 → 8) | |
| | kochgeschrumpft | |
| | Durchmesser | 4,6 +/- 1,6 |
| | von 3 auf 6 mm | n = 26 |
| | (zweistufig 3 → 5 → 6) | |
| | kochgeschrumpft | |
| | Durchmesser | 2,3 +/- 0,6 |
| | von 5 auf 8 mm | n = 13 |
| | (einstufig) | |
| | nicht kochgeschrumpft | |
| | Durchmesser | 3,5 +/- 1,0 |
| | von 3 auf 6 mm | n = 13 |
| | (zweistufig 3 → 5 → 6) | |
| | nicht kochgeschrumpft | |
| Trikot-Atlas N | Durchmesser | 4,9 +/ 0,4 |
| | von 6 auf 6 mm | n = 13 |
| | (einstufig) | |
| | kochgeschrumpft | |
| | Durchmesser | 4,4 +/- 0,4 |
| | von 6 auf 6 mm | n = 13 |
| | (einstufig) | |
| | nicht kochgeschrumpft | |
| Trikot-Atlas N2 | Durchmesser | 5,9 +/- 0,4 |
| | von 6 auf 6 mm | n = 13 |
| | (einstufig) | |
| | kochgeschrumpft | |
| | Durchmesser | 4,6 +/- 0,4 |
| | von 6 auf 6 mm | n = 13 |
| | (einstufig) | |
| | nicht kochgeschrumpft | |

### Beispiel 7

### Compliance von ummantelten Venen

Zur Püfung der Compliance von Venen mit Ummantelungen wurden Jugularvenen vom Schaf ummantelt mit verschiedenen Netzschläuchen aus Polyester (Dacron) bei einer Strömungsgeschwindigkeit von 300 ml/min und einer Druckamplitude von 50 mmHg bei verschiedenen Druckwerten auf ihre dynamische Umfangscompliance und den Durchmesser untersucht. Die Messwerte wurden mit nativen Karotisarterien und Jugularvenen vom Schaf sowie Gefäßersatz aus Polytetrafluorethylen (PTFE) verglichen.

Die Durchmesser von Jugularvenen vom Schaf betrugen 14,7 ± 2,92 mm und die Umfangscompliance 2,78 ± 1,4 %/100 mmHg. Durchmesser von Karotisarterien vom Schaf betrugen 6,6 ± 0,27 mm und die Umfangscompliance 3,3 ± 0,9 %/100 mmHg. Die Umfangscompliance von PTFE-Gefäßersatz betrug 0,06 ± 0,05 %/100 mmHg. Der externe Stentdurchmesser reduzierte sich auf mittlere Werte von 7,4 ± 0,12 und entspricht somit nahezu dem Arteriendurchmesser. In Abhängigkeit von der Struktur des Stents betrug die Umfangscompliance von Venen mit Stents von 1,98 bis 0,74 %/100 mmHg.

Die gemäß der Erfindung mit einer textilen Konstruktion ummantelte Vene zeigte eine nichtlineare Compliance, wie sie auch bei natürlichen Blutgefäßen und speziell bei Arterien gefunden wird. Es kann eine lange Wirksamkeit und Lebensdauer der ummantelten nativen Gefäßprothesen erwartet werden, wobei auch die Intimahyperplasie verringert ist.

## Patentansprüche

1. Ummantelung für die Verstärkung von natürlichen Venen zur Verwendung als chirurgisches Implantat in Form einer Netzstruktur in textiler Konstruktion, **dadurch gekennzeichnet, dass** sie nahtlos rohrförmig als im wesentlichen velourfreies Gewirk ausgebildet ist und hochporöse Netzöffnungen im wesentlichen in Gestalt von Polygonen aufweist und mit einer Reihendichte pro Zentimeter von 15 bis 40 sowie mit einer Stäbchendichte pro Zentimeter von 15 bis 30 ausgebildet ist.

2. Ummantelung nach Anspruch 1, **dadurch gekennzeichnet, dass** sie durch Wirken in Einfach-Trikotbindung ausgebildet ist.

3. Ummantelung nach Anspruch 1, **dadurch gekennzeichnet, dass** sie durch Wirken in kombinierten Bindungstechniken, insbesondere in Trikot-Atlasbindung ausgebildet ist.

4. Ummantelung nach einem der vorhergehenden Ansprüche, **dadurch gekennzeichnet, dass** sie im wesentlichen aus biokompatiblen Polymerfasern gebildet ist.

5. Ummantelung nach einem der vorhergehenden Ansprüche, **dadurch gekennzeichnet, dass** sie im wesentlichen frei von texturierten Fäden ausgebildet ist.

6. Ummantelung nach einem der vorhergehenden Ansprüche, **dadurch gekennzeichnet, dass** sie aus Multifilamentgarn gebildet ist.

7. Ummantelung nach einem der vorhergehenden Ansprüche, **dadurch gekennzeichnet, dass** sie aus Garn mit 2 bis 500 Filamenten, insbesondere 5 bis 250 Filamenten, bevorzugt 10 bis 100 Filamenten gebildet ist.

8. Ummantelung nach einem der vorhergehenden Ansprüche, **dadurch gekennzeichnet, dass** sie aus Garn mit einer Feinheit von 50f40 dtex gebildet ist.

9. Ummantelung nach einem der vorhergehenden Ansprüche, **dadurch gekennzeichnet, dass** sie mit einer Maschenweite von 100 bis 1000 µm, insbesondere 300 bis 600 µm ausgebildet ist.

10. Ummantelung nach einem der vorhergehenden Ansprüche, **dadurch gekennzeichnet, dass** die innere Begrenzung der Netzöffnungen abgerundet, insbesondere wabenförmig ausgebildet ist.

11. Ummantelung nach einem der vorhergehenden Ansprüche, **dadurch gekennzeichnet, dass** sie mit einer Wanddicke von 0,05 bis 0,5 mm, insbesondere 0,15 bis 0,25 mm ausgebildet ist.

12. Ummantelung nach einem der vorhergehenden Ansprüche, **dadurch gekennzeichnet, dass** sie mit einem Nenndurchmesser von 2 bis 15 mm ausgebildet ist.

13. Ummantelung nach einem der vorhergehenden Ansprüche, **dadurch gekennzeichnet, dass** sie durch thermisches Schrumpfen vorbehandelt ist.

14. Ummantelung nach einem der vorhergehenden Ansprüche, **dadurch gekennzeichnet, dass** sie thermofixiert ist.

15. Ummantelung nach einem der vorhergehenden Ansprüche, **dadurch gekennzeichnet, dass** sie weitgehend formstabil ist.

16. Ummantelung nach einem der vorhergehenden Ansprüche zur Verwendung als Verstärkung von Venen in der Humanmedizin und Veterinärmedizin.

## Claims

1. Sheathing for reinforcing natural veins for use as a surgical implant in the form of a net structure in textile construction, **characterized in that** it is formed as a seamless tube in the form of an essentially pile-free formed-loop knit and has highly porous apertures in the net which are essentially in the shape of polygons and is formed with a course density per centimetre in the range from 15 to 40 and also with a wale density per centimetre in the range from 15 to 30.

2. Sheathing according to Claim 1, **characterized in that** it is formed by formed-loop knitting with single tricot lapping.

3. Sheathing according to Claim 1, **characterized in that** it is formed by formed-loop knitting with combined lapping techniques, in particular with tricot-atlas lapping.

4. Sheathing according to any one of the preceding claims, **characterized in that** it is essentially formed from biocompatible polymeric fibres.

5. Sheathing according to any one of the preceding claims, **characterized in that** it is essentially free of textured strands.

6. Sheathing according to any one of the preceding claims, **characterized in that** it is formed from multifilament yarn.

7. Sheathing according to any one of the preceding claims, **characterized in that** it is formed from yarn having 2 to 500 filaments, in particular 5 to 250 filaments, preferably 10 to 100 filaments.

8. Sheathing according to any one of the preceding claims, **characterized in that** it is formed from 50f40 dtex yarn.

9. Sheathing according to any one of the preceding claims, **characterized in that** it has a mesh size of 100 to 1000 µm, in particular 300 to 600 µm.

10. Sheathing according to any one of the preceding claims, **characterized in that** the inner boundary of the net apertures is rounded off, in particular honeycomb-shaped.

11. Sheathing according to any one of the preceding claims, **characterized in that** it has a wall thickness of 0.05 to 0.5 mm, in particular 0.15 to 0.25 mm.

12. Sheathing according to any one of the preceding claims, **characterized in that** it has a nominal diameter of 2 to 15 mm.

13. Sheathing according to any one of the preceding claims, **characterized in that** it is pretreated by thermal shrinking.

14. Sheathing according to any one of the preceding claims, **characterized in that** it is heat-set.

15. Sheathing according to any one of the preceding claims, **characterized in that** it is substantially shape-stable.

16. Sheathing according to any one of the preceding claims for use as reinforcement of veins in human medicine and veterinary medicine.

## Revendications

1. Gaine destinée à renforcer des veines naturelles et à être utilisée comme implant chirurgical, qui présente la forme d'une structure en treillis textile, **caractérisée en ce que**
elle est configurée en forme tubulaire sans couture, en tricot essentiellement exempt de velours, elle présente des ouvertures de treillis à haute porosité essentiellement en forme de polygones et elle est configurée à une densité de ligne par centimètre de 15 à 40 ainsi qu'à une densité de traverse par centimètre de 15 à 30.

2. Gaine selon la revendication 1, **caractérisée en ce qu'**elle est formée par tricotage en armure tricotée simple.

3. Gaine selon la revendication 1, **caractérisée en ce qu'**elle est configurée par tricotage par des techniques de liaison combinée, en particulier en armure tricotée atlas.

4. Gaine selon l'une des revendications précédentes, **caractérisée en ce qu'**elle est formée essentiellement de fibres polymères biocompatibles.

5. Gaine selon l'une des revendications précédentes, **caractérisée en ce qu'**elle est réalisée essentiellement sans fil texturé.

6. Gaine selon l'une des revendications précédentes, **caractérisée en ce qu'**elle est formée de fils multifilaments.

7. Gaine selon l'une des revendications précédentes, **caractérisée en ce qu'**elle est formée de fils comptant de 2 à 500 filaments, en particulier de 5 à 250 filaments et de préférence de 10 à 100 filaments.

8. Gaine selon l'une des revendications précédentes, **caractérisée en ce qu'**elle est formée de fils d'une finesse de 50f40 dtex.

9. Gaine selon l'une des revendications précédentes, **caractérisée en ce qu'**elle est formée à une maille de 100 à 1000 µm et en particulier de 300 à 600 µm.

10. Gaine selon l'une des revendications précédentes, **caractérisée en ce que** la frontière intérieure des ouvertures du treillis est arrondie et en particulier a la forme d'un nid d'abeilles.

11. Gaine selon l'une des revendications précédentes, **caractérisée en ce qu'**elle est formée à une épaisseur de paroi de 0,05 à 0,5 mm et en particulier de 0,15 à 0,25 mm.

12. Gaine selon l'une des revendications précédentes, **caractérisée en ce qu'**elle est formée à un diamètre nominal de 2 à 15 mm.

13. Gaine selon l'une des revendications précédentes, **caractérisée en ce qu'**elle est prétraitée par retrait thermique.

14. Gaine selon l'une des revendications précédentes, **caractérisée en ce qu'**elle est fixée thermiquement.

15. Gaine selon l'une des revendications précédentes, **caractérisée en ce que** sa forme est largement stabilisée.

16. Gaine selon l'une des revendications précédentes, destinée à être utilisée comme renfort de veines en médecine humaine et en médecine vétérinaire.
